# EUROPEAN PATENT APPLICATION

(11) **EP 4 270 004 A1**
(43) Date of publication of application: **01.11.2023**
(21) Application number: 23165934.3
(22) Date of filing: 31.03.2023
(51) Int. Cl.: G01N 33/00, H01M 10/42, H01M 6/50, B60L 58/10

(54) **TIERED GAS MONITORING FOR BATTERY FAILURES**

(30) Priority: 27.04.2022 US 202217730892
(71) Applicant: Analog Devices International Unlimited Company, Limerick (IE)
(72) Inventor: PONOMAREV, Youri Victorovitch, 3110 Rotselaar (BE); OPPENHAEUSER, Joern, 61440 Oberursel (DE)
(74) Representative: Yang, Shu

(57) **Abstract**

A gas monitoring system can include a chemical concentration secondary gas sensor, with a conductive heater element, which can be heated to a standby temperature slightly above an ambient temperature level of a gaseous ambient environment. A thermal characteristic of the environment can be monitored by a thermal property primary sensor and configured to provide a triggering output in response to a physical characteristic of the conductive heating element meeting at least one criterion. In response to the triggering output, the heater is controllable to heat the chemical concentration secondary gas sensor to a secondary operating temperature at which the chemical concentration secondary gas sensor can detect the presence of a specified gas in the environment.

## Description

### TECHNICAL FIELD

This document pertains generally, but not by way of limitation, to monitoring gas concentration in an environment, such as in an electric vehicle or other battery environment.

### BACKGROUND

A battery can include a battery cell or a battery module, either of which can be included in a battery pack or a battery compartment. It is desirable to monitor battery health such as to anticipate or detect thermal runaway accompanying battery failure. Early detection is helpful because a thermal runaway event can lead to a battery fire or explosion. In automotive applications, early detection and alerting of signs of battery failure or thermal runaway can help allow a passenger to timely exit a vehicle to reach safety.

### SUMMARY

Battery health can be monitored at the battery cell level, at a battery module level, or at a battery pack level, such as by using a temperature sensor in a battery compartment to detect a thermal runaway event. While one approach to monitoring battery health can include using a pressure sensor to measure pressure within a battery compartment, a battery pack will generally include a pressure equalization vent, which can make slow pressure changes hard to detect, such that pressure-based battery failure detection can be limited to being later than desirable in the failure progression when stronger pressure changes occur. Such a pressure sensor can also be affected by unrelated events as well as being difficult to use for detecting battery failure before the start of a thermal runaway event.

Battery health can also be monitored using one or more gas sensors to monitor gas composition in the battery environment, such as within a battery compartment headspace. An optical non-dispersive infrared (NDIR) sensor can be used for gas detection, such as to detect CO2 or CO. However, CO2 and CO may only appear at that late stage of battery failure at which thermal runaway is fairly immediately imminent. Battery health monitoring via volatile gas sensing can use one or more Metal oxide sensors to detect battery failure at an earlier stage of the failure progression. However, metal oxide sensors have limited longevity. In operation, the heated metal oxide layer of a metal oxide sensor can become contaminated. Such contamination can significantly change the sensor response, rendering the sensor unusable, particularly in demanding applications. For example, automotive and industrial batteries may require a usable lifetime of more than 10 years.

The present inventors have recognized, among other things, the need for a robust approach to monitoring battery health that is not susceptible to external influence, and the need for an approach to monitoring battery health that can detect battery failure early in the battery failure progression, such as before the onset of thermal runaway event. The present techniques can help provide a solution to this problem. The present techniques can include monitoring a thermal property of a gas in an environment for one or more changes potentially indicative of battery health. When a primary change indicator is met by monitoring the thermal property, then a secondary change indicator can be measured and compared to at least one criterion. For example, a secondary gas sensor can be switched on or into an operating mode and used to further determine, based on a measured concentration of a gas composition component, whether the primary change indicator does indeed rise to being a sign of battery failure. Similar gas monitor techniques can also be useful in applications other than automotive battery health monitoring.

This overview is intended to provide an overview of subject matter of the present patent application. It is not intended to provide an exclusive or exhaustive explanation of the invention. The detailed description is included to provide further information about the present patent application.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1 is a perspective view showing an example of a sensor that can be located near a battery cell, battery module, or battery pack, for example, such as can be located in the headspace of a battery compartment.
FIG. 2 is a side view of an example of a configuration of the gas monitoring system.
FIG. 3 is a cross section of an example of a metal oxide gas concentration sensor.
FIG. 4 is a perspective view of an example of a metal oxide gas concentration sensor.
FIG. 5 is a diagram of a "hot-wire" method for measuring one or more thermal properties.
FIG. 6 is a block diagram of an example of the system that may be configured using one or more monolithically integrated gas monitoring system.

### DETAILED DESCRIPTION

This document describes, among other things, techniques that can include a gas monitoring system for monitoring gas in a battery compartment, such as can be associated with a battery pack, battery module, battery cell, or other environment of interest. The system can include a thermal property primary gas sensor, such as to detect a thermal property of an environment, such as thermal conductivity of the environment, a heat capacity of the environment, or a thermal diffusivity of the environment. The primary gas sensor can be configured to provide a triggering output in response to a determined change in a thermal property of the environment, from which a change in a gas composition of the environment can be inferred. Then, in response to the triggering output from the thermal property primary gas sensor, a chemical concentration secondary gas sensor (such as a metal oxide ("MOx") chemical concentration sensor) can be enabled, such as for being placed into an operating mode for detecting an amount of a specified first gas component in the environment.

Examples of one or more thermal properties detectable by the primary gas sensor can include, among other things, a thermal conductivity of the environment, a heat capacity of the environment, or a thermal diffusivity of the environment-one or more of which may vary with the gas composition of the environment. In a particular example, an electrical resistance or conductivity of a heater element associated with the secondary gas sensor can be used as the thermal property of the primary gas sensor. The primary sensor can trigger enabling of the second sensor, such as by elevating the temperature of a heater element of the secondary sensor from a standby temperature to an operating temperature at which the secondary sensor can detect a concentration of a first gas component in the environment.

### Battery Monitoring

For battery monitoring, a nearby gas composition, such as in the headspace of a battery compartment, can be monitored to help with early detection of battery failure. This can help to make a battery safer by allowing early detection of a potential catastrophic failure. Earlier detection of a potential catastrophic failure may help a battery management or other system to react to the detected event, such as to start responding with a corrective action or to notify the user of the potential failure. Corrective action can include disconnecting or discharging an affected battery compartment, increasing cooling to counteract heating that may be leading to a thermal runaway event, or limiting demand on the battery, one or more of which may help to halt or reverse the battery failure or thermal runaway. For example, the gas monitoring system can be used to detect one or more volatile gases that may indicate imminent failure of a battery in the headspace of a battery compartment.

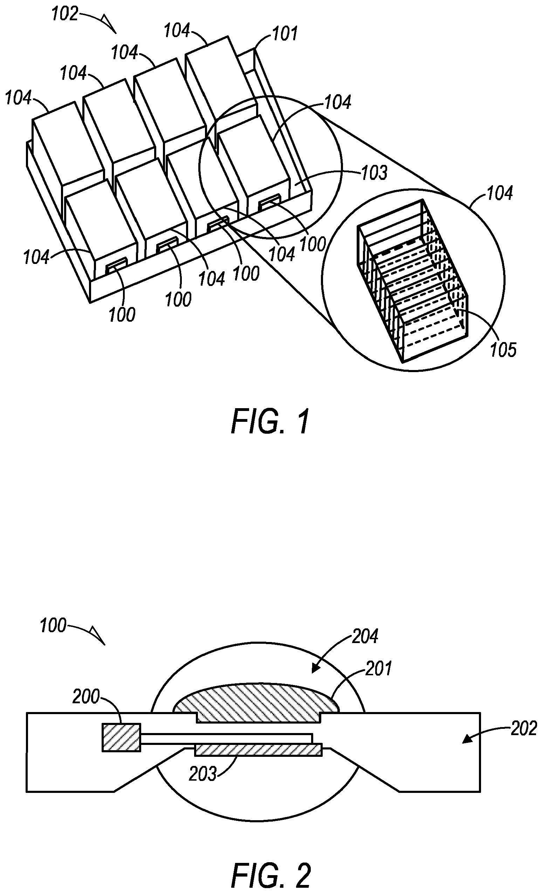

FIG. 1 depicts an example of an arrangement of a gas monitoring system such as for battery monitoring. The gas monitoring system 100 may be located inside or near a battery compartment 101, such as near a battery pack 102, a battery module 104, or a battery cell 105. For example, the gas monitoring system 100 can be located within a battery compartment 101, such as in the headspace 103 of the battery compartment 101. As a battery progresses through failure stages, the battery may generate volatile gases having a composition dependent on and indicative of the stage of battery failure.

A battery at an early stage of failure, may primarily produce, in the case of electrolyte leakage, gases such as Ethylene Carbonate ("EC"), Dimethyl Carbonate ("DMC"), Diethyl Carbonate ("DEC"), and Ethyl Methyl Carbonate ("EMC"). In the case of electrolysis of water, a battery may primarily produce gases such as H2 and O2. A battery at a later stage of battery failure, the so called "first venting", may primarily produce gases such as DEC, DMC, EMC, and H2O, as well as producing lesser amounts of gases such as CO, CO2, EC, and C4H10. A battery at a stage of battery failure where combustion is fairly immediately imminent, the so called "thermal runaway", may primarily produce gases such as CO2, CO, C2H4, and H2 as well as lesser portions of gases such as C2H2, C2H6, CH4, DEC, DMC, EMC, H2O, C4H10, and O2. A battery that has combusted may primarily produce gases such as CO, CO2, and HF. [*see,* Essl, Christiane, Lauritz Seifert, Michael Rabe, and Anton Fuchs. "Early Detection of Failing Automotive Batteries Using Gas Sensors". Batteries 7, no. 2 (June 2021): 25. https://doi.org/10.3390/batteries7020025.]

These volatile gases may be detectable in an ambient gas environment 204, such as in the headspace 103 of the battery compartment 101. Gases, such as those generated by electrolytes such as Ethylene Carbonate, Dimethyl Carbonate, Diethyl Carbonate, or Ethyl Methyl Carbonate may be produced at an early stage of battery failure. A change in gas composition of the ambient gas environment 204 due to the addition of a volatile gas can cause a change in a thermal property of the ambient gas environment 204, such as if a thermal property of the volatile gas is significantly different from the ambient gas environment 204, before adding the volatile gas. Quite a number of volatile gases may have a thermal property that is significantly different from that same thermal property of air.

Table 1 below lists thermal conductivity, specific heat capacity, thermal diffusivity, and density of Air, N₂, CO₂, O₂, H₂, He, and CH₄. (*see,* Kliche, Kurt, et al. "Sensor for thermal gas analysis based on micromachined silicon-microwires." IEEE Sensors Journal 13.7 (2013): 2626-2635.) Table 1 shows that thermal conductivity, thermal diffusivity, or heat capacity may be used for selective sensing of a particular gas in a mixture. In addition, the inventors have observed experimentally that the thermal properties of a target volatile battery gas to be detected, e.g., DMC electrolyte volatiles may have thermal properties that can be different enough from the thermal properties of ambient air such that, at the detection concentrations of interest, changes in such thermal properties can be used to infer changes in concentration of various gas components in the ambient air in the environment of the battery space. Thus, one or more thermal properties can be used to detect the presence of DMC electrolyte volatiles and other volatile battery gas components of interest-at least to a degree helpful to trigger a more accurate secondary gas concentation sensor that need not rely on inferred gas concentration from thermal properties in the environment. For example, such a secondary gas concentration sensor can directly identify concentration of a target gas component in the ambient air of the environment near the battery. As an illustrative example, a gas component (e.g., CO₂) having at least about a 30% difference from air in a specified thermal property can be detected at concentrations of interest using a primary thermal property gas concentration sensor, and then more accurately determined using a more accurate secondary gas concentration sensor than the primary thermal property gas concentration sensor.

**Table 1.**

| THERMAL PROPERTIES OF SOME GASES AT PRESSURE *ρ* = 1 BAR AND TEMPERATURE *T* = 25 °C | | | | | | | | |
|---|---|---|---|---|---|---|---|---|
| | Thermal conductivity λ (mW m⁻¹K⁻¹) | | Specific Heat Capacity *Cp* (kJ kg⁻¹K⁻¹) | | Thermal Diffusivity *a* (mm²s⁻¹) | | Density *ρ* (kg m⁻³) | |
| Air | 26.2 | diff. (%) | 1.01 | diff. (%) | 22.3 | diff. (%) | 1.17 | diff. (%) |
| N₂ | 25.9 | 1.2 | 1.04 | 3.0 | 22.0 | 1.4 | 1.13 | 3.4 |
| CO₂ | 16.6 | 37 | 0.85 | 16 | 11.0 | 51 | 1.78 | 52 |
| O₂ | 26.4 | 0.7 | 0.92 | 8.9 | 21.9 | 1.8 | 1.29 | 10 |
| H₂ | 181 | 591 | 14.3 | 1316 | 154 | 591 | 0.08 | 93 |
| He | 154 | 488 | 5.19 | 414 | 185 | 730 | 0.16 | 86 |
| CH₄ | 34.0 | 30 | 2.22 | 120 | 23.6 | 5.8 | 0.65 | 44 |

Therefore, the ambient gas environment 204 in the headspace 103 of the battery compartment 101 may be monitored for a change in one or more thermal properties indicating battery failure or thermal runaway.

FIG. 2 shows a side view of an example of portions of a possible configuration of the gas monitoring system 100. In FIG. 2, the gas monitoring system 100 can include a combination of a thermal property primary sensor 200, such as a "hot-wire", "three-omega", or other thermal property sensor 200 and a gas concentration secondary sensor 201. The gas monitoring system 100 can also include a structural member 202, such as a base, substrate, package, or the like, and a heater element 203. The heater element 203 can be configured as a part of either or both of the thermal property primary sensor 200 and the gas concentration secondary sensor 201. The thermal property primary sensor 200 can detect a change in a thermal property of the ambient gas environment 204, which can be used to trigger an enabling of the gas concentration secondary sensor 201. The thermal property primary sensor 200 may be configured to detect a change in a thermal property of the ambient gas environment 204 by monitoring the heater element 203, such as by measuring the electrical resistance of the heater element 203 and using the "hot-wire" method. The gas concentration secondary sensor 201 may then be used to help verify battery failure. The chemical concentration secondary sensor 201 may help verify battery failure by determining whether a volatile gas, which indicates battery failure, is present in the environment. Such verification can help to prevent false reports of battery failure. The gas monitoring system 100 may be configured with a Metal Oxide ("MOx") or other gas concentration sensor as the gas concentration secondary sensor 201.

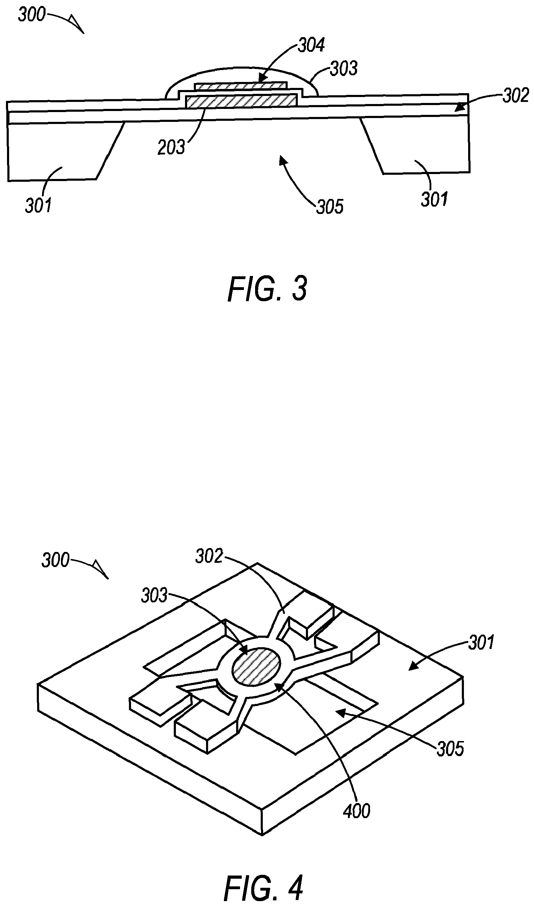

FIG. 3 and FIG. 4 show an example of a MOx gas concentration sensor. Briefly, a MOx gas concentration sensor can include a chemresistor with an electrical resistance that is a function of the chemical composition of an ambient gas environment. A MOx gas concentration sensor may be formed with a transitional metal oxide semiconductor material that may be heated to around 280 - 460°C during gas concentration detection operation. A MOx gas concentration sensor can be cost effective, can have high sensitivity, and can have a fast non-specific response to one or more oxidizing or reducing gases. However, the MOx layer of a MOx gas concentration sensor can become contaminated while it is heated to its operating temperature. Therefore, in prolonged operation, the response of the MOx gas concentration sensor can change significantly over time, reducing its reliability. At temperatures below 100⁰C, contamination of the MOx layer can be reduced significantly, and the power consumption of the MOx gas concentration sensor can also be reduced when only heating the MOx sensor to such lower temperature. However, the sensitivity of a MOx gas concentration sensor drops significantly at lower temperatures and can be unusable at temperatures below 100⁰C. A MOx gas concentration sensor 300 may include a substrate 301. The substrate 301 may be connected by a hotplate member 302 to support a hotplate 400, including a heater element 203. The hotplate member 302 can also support a metal oxide layer 303, and a metal oxide sensing electrode 304. The hotplate member 302 can help to thermally isolate the metal oxide layer 303, the metal oxide sensing electrode 304, and the heater 203 from the substrate 301. Such thermal isolation can include creating a thermal isolation cavity 305, which can be capable of receiving ambient or other gas from an ambient gas environment 204, for example, such as a headspace 103 of a battery compartment 101 or other environment. The hotplate 400 and the heater element 203 may be arranged to heat the metal oxide sensing electrode 304 and the metal oxide layer 303. Once heated, the metal oxide sensing electrode 304 can be used to measure an electrical resistance or conductivity of the metal oxide layer 303, which will change as a function of the extent to which the metal oxide layer 303 has been oxidized. This is because the oxidation of the metal oxide layer 303 is dependent on the concentration of a specified gas component in an ambient gas environment 204 surrounding the metal oxide layer 303.

In FIG. 2, the gas concentration secondary sensor 201 can be configured as a MOx gas concentration sensor 300, the structural member 202 may include the substrate 301, the hotplate member 302, and/or the hotplate 400 of the MOx gas concentration sensor 300. The thermal isolation cavity 305 of the MOx gas concentration sensor 300 can be used to suspend or otherwise separate or isolate the heater element 203 from other structural components. The thermal isolation cavity 305 can allow entry of one or more gases from the ambient gas environment 204 of the headspace 103 of the battery compartment 101 into the thermal isolation cavity 305. In this way, a change in resistance or conductance of the heater element 203 can be better correlated to changes in gas component concentrations of the nearby ambient gas environment to be analyzed, and less affected by heat sinking or heat sourcing properties of the structure to which the heater element 203 is attached. The thermal isolation cavity 305 allows more efficient thermal transfer to the nearby ambient gas environment, for accurate and lower-power determination of ambient gas concentration via measurement in changes in thermal properties of the ambient gas environment. For example, the thermal property primary sensor 200 can include impedance or conductance measurement circuitry, which can be configured to monitor the electrical resistance or conductivity of the heater element 203 that is associated with one or both of the primary gas sensor or the secondary gas sensor and to use the "hot-wire method" to determine one or more thermal properties of the environment.

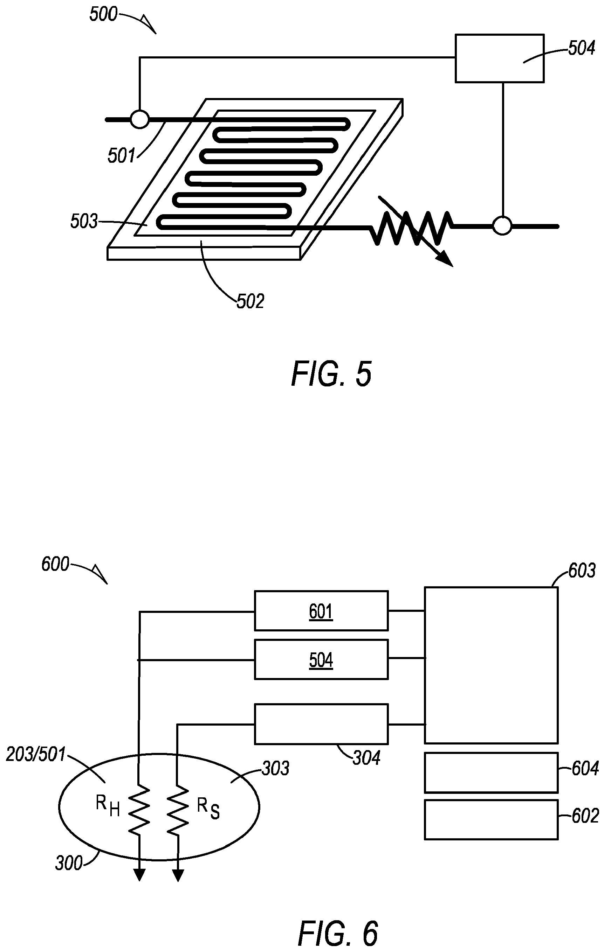

FIG. 5 shows an example of a cross section of a "hot-wire" thermal conductivity sensor. Briefly, a "hot-wire" thermal conductivity sensor can use a heated conductor or "hot-wire". Electrical resistance or conductivity of the heated wire can be monitored. Heat from the hot-wire can be thermally conducted away from the wire into the environment in a manner that depends on one or more thermal properties of the environment, such as its thermal conductivity, its heat capacity, or its thermal diffusivity, which can be affected by a gas concentration or other composition of the environment. By measuring an electrical conductivity of the wire, which is also a function of a temperature of the heated wire, one or more thermal characteristics of the environment (e.g., thermal conductivity) indicative of a gas or other composition of the environment can be inferred. In an example, a "hot-wire" thermal conductivity sensor 500 may include a heated conductor 501, which may be held in place by an insulated layer 502 and exposed to a specimen 503, and a electrical resistance sensor 504 . The electrical resistance sensor 504 may be attached to the heated conductor 501 to monitor the resistance of the heated conductor 501. When the thermal conductivity of the specimen 503 changes, such as due to a change in the composition of the specimen 503, the rate of heat removal from the heated conductor 501 may change. This, in turn, may result in a change of resistance in the heated conductor 501. The change in resistance can then be measured by the electrical resistance sensor 504.

In FIG. 2, for a battery monitoring system, the gas monitoring system 100 may include a MOx gas concentration sensor 300 and a thermal property primary sensor 200 configured as a "hot-wire" thermal conductivity sensor that may include the heater element 203 of the MOx gas concentration sensor 300 configured as the heated conductor 501 of the thermal conductivity sensor 500. The electrical resistance sensor 504 can be configured to measure the resistance of the heater element 203, and the ambient gas environment 204 of the headspace 103 of the battery compartment 101 as the specimen 503 of the thermal conductivity sensor 500. The structural member 202 of the gas monitoring system 100 may be configured as the electrically insulated layer 502 of the thermal conductivity sensor 500. The heater element 203 can be configured as a "hot-wire" thermal conductivity sensor 500, which may be operated such that the heater element 203 can be heated to a standby temperature that is slightly above ambient temperature, such as approximately 20 - 100 Kelvin above an ambient temperature. The range of an ambient temperature of a battery in an electric vehicle may include temperatures from around -30°C to around 60°C. The electrical resistance sensor 504 can monitor the electrical resistance of the heater element 203 and is continuously or recurrently polled to detect changes to a thermal property of the ambient gas environment 204, such as one or more of its thermal conductivity, its heat capacity, or its thermal diffusivity. The data provided by the electrical resistance sensor 504 can be processed by interface circuitry to determine whether possible signs of battery failure are present. If so, a triggering output can be sent to increase the temperature of the heater element 203 from the standby temperature to an operating temperature of the MOx gas concentration sensor 300. The operating temperature of a MOx gas concentration sensor 300 may be around 280 - 460⁰C. When enabled by the increase in temperature to the operating temperature, the MOx gas concentration sensor 300 can determine if a specified gas component is present in the ambient gas environment 204. If the specified gas component is present, the gas monitoring system 100 may produce an output, such as sending a signal to alert a user or a battery management or other system component to take corrective action. If the specified gas component is not present, the gas monitoring system 100 can disable the enabling system, and return to monitoring thermal properties using the primary sensor, such as by returning the heater element 203 to a standby temperature and resuming continuously or recurrently polling the electrical resistance sensor 504.

Such techniques can offer several benefits. Using multiple measurement principles provides built-in redundance. This can help inhibit or prevent potential false readings. By monitoring the heater element 203 at a temperature below 100⁰C, the gas monitoring system 100 can help increase the lifespan of the system significantly over that of a MOx gas concentration sensor 300 alone. This is because the MOx gas concentration sensor 300 can generally be kept below the operating or other temperature at which the MOx layer is significantly contaminated until the thermal property primary sensor 200 provides an initial indication of a potential concern for battery failure, at which time the secondary sensor can be engaged, but not otherwise. The gas monitoring system 100 may also help reduce overall power consumption significantly compared to a MOx gas concentration sensor 300 alone, as the power consumption of a MOx gas concentration sensor 300 depends on the amount of heating used to provide the (standby or operating) temperature of the MOx gas concentration sensor 300.

### Other Configurations

Other examples of the gas monitoring system 100 can be configured to use a different type of primary sensor 200 than a "hot-wire" thermal property sensor 200, such as a sensor using the "three-omega" method, or a different type of gas concentration secondary sensor 201 than a MOx gas concentration sensor 300. Other examples of the gas monitoring system 100 can include a thermal property primary sensor 200 and a gas concentration secondary sensor 201 that do not share a heater element 203, or are not incorporated into a single device. The gas monitoring system 100 may be configured as a system in package or fully monolithically integrated. The gas monitoring system 100 may also be implemented using separate, discrete sensors and other electronic components.

The gas monitoring system 100 may include one or more additional or alternative primary or secondary sensors. For example, an additional gas concentration secondary sensor may be configured to detect the same or different gas species or concentrations as the gas concentration secondary sensor 201. The additional secondary sensor may include a MOx layer 303 configured with a different composition than the first MOx gas concentration sensor 300. This can help provide additional functionality or flexibility in selecting a specified gas component to monitor, which may help provide improved selectivity. The gas monitoring system 100 may include one or more additional auxiliary thermal property or other primary sensors. The one or more additional thermal property sensor may be configured to monitor the same or different thermal property as the thermal property primary sensor 200. The gas monitoring system 100 may include an NDIR sensor configured as a primary or secondary sensor.

Enabling a gas concentration secondary sensor 201 may include increasing the temperature of the gas concentration secondary sensor 201 to an operating temperature at which gas concentration detection operation is possible, or the powering on of the gas concentration secondary sensor 201.

The gas monitoring system 100 may include one or more ambient environmental condition sensors such as can be configured to monitor an ambient environmental condition (other than gas composition concentration), such as ambient temperature, humidity, flow, or barometric pressure, such as for use in combination with the thermal property primary sensor 200 and gas concentration secondary sensor 201 to help improve the accuracy of the gas monitoring system 100.

The gas monitoring system 100 may include one or more pairs of thermal property primary sensor 200, which may include at least a first thermal property primary sensor 200 and a second thermal property primary sensor 200 that have been paired together, and one or more pairs of gas concentration secondary sensors 201, which may include at least a first gas concentration secondary sensor 201 and a second gas concentration secondary sensor 201 that have been paired together. The first thermal property primary sensor 200 and first gas concentration secondary sensor 201 can be configured to monitor the environment of interest, such as the headspace 103 of a battery compartment 101. The second thermal property primary sensor 200 and the second gas concentration secondary sensor 201 can be placed in a environment with a known and unchanging gas composition, such as an enclosure filled with a known gas. The gas monitoring system 100 can compare the measurement, such as by performing a differential measurement, of the first thermal property primary sensor 200 and the first gas concentration secondary sensor 201 to the measurements of the second thermal property primary sensor 200 and the second gas concentration secondary sensor 201. The gas monitoring system 100 can also use the first thermal property primary sensor 200 and second thermal property primary sensor 200 for Differential Scanning calorimetry, which can help accurately determine the specific heat capacity of an ambient gas environment 204.

### Other Use-Cases

The gas monitoring system 100 may be used for other volatile gas monitoring applications such as hydrogen, methane, or difluoromethane leak detectors. The gas monitoring system 100 may also be used for applications such as creating an accurate air quality sensor, such as for populated environments.

### Component specifications

Using primary sensor heater resistance measurements with a resolution of 22 bits or better can help ensure that measurements are sufficiently sensitive. Total analog-to-digital converter resolution requirements can be reduced by using one or more offset compensation techniques in the overall signal chain.

A thermal resistance of the heater element being above 10,000 K/W can help ensure reliable function of a "hot-wire" thermal conductivity sensor.

Configuring the system in the smallest reasonable form factor may help reduce the cost of the system. For example, FIG. 6 depicts an example of the system that may be configured using one or more monolithically integrated gas monitoring system configured with a MOx gas concentration sensor 300 and a thermal property primary sensor 200, such as can be integrated on the same integrated circuit (IC) 600 with control circuitry 601 and sensor signal processing circuitry 603, such as can be configured to monitor the headspace 103 of a battery pack 102, and can include sufficient on-board or other computing functionality for a feature extraction from the data. Such computing functionality can be provided by one or more of a microcontroller 604, an Analog Front End (AFE) circuit, a Digital Signal Processor circuit, non-volatile or volatile memory circuitry, and wired or wireless external communication transceiver circuitry 602 . The thermal property primary sensor 200 can be a "hot-wire" thermal conductivity sensor 500 that is configured to use the heater element 203 of the MOx gas concentration sensor 300 as the heated conductor 501 of the thermal property primary sensor 200. The MOx gas concentration sensor 300 may include a metal oxide layer 303 a and metal oxide sensing electrode 304. The thermal property primary sensor can include an electrical resistance sensor 504 configured to monitor the electrical resistance of the heater element 203. At least some of the signal-processing or other functionality can be carried out at least partially remotely, such as through Cloud computing.

### Numbered Aspects

By way of non-limiting example, some aspects of the disclosure are set out in the following numbered clauses.

Numbered Clause 1. A gas monitoring system for monitoring gas in an environment, the system comprising:
a thermal property primary gas sensor, configured to provide a triggering output in response to determining a change in a thermal property of the environment indicating a change in a gas composition of the environment; and
a chemical concentration secondary gas sensor, enabled in response to the triggering output from the thermal property primary gas sensor, for detecting an amount of a specified first gas component in the environment.

Numbered Clause 2. The system of Numbered Clause 1, wherein the chemical concentration secondary gas sensor is enabled by at least one of powering up the chemical concentration secondary gas sensor or heating a heater element of the chemical concentration secondary gas sensor to an operating temperature at which the chemical concentration secondary gas sensor is capable to detect an amount of the specified first gas component in the environment.

Numbered Clause 3. The system of Numbered Clause 2, wherein the chemical concentration secondary gas sensor is at least one of powered down or operated at a standby temperature by the heater element in an absence of being enabled in response to the triggering output from the thermal property primary gas sensor, wherein the standby temperature is less than the operating temperature.

Numbered Clause 4. The system of any preceding Numbered Clause, wherein the thermal property primary gas sensor is configured to detect a thermal property comprising at least one of a thermal conductivity of the environment, a heat capacity of the environment, or a thermal diffusivity of the environment, wherein the thermal property varies with a gas composition of the environment.

Numbered Clause 5. The system of any preceding Numbered Clause, further comprising interface circuitry, receiving the triggering output from the thermal property primary gas sensor, and providing a responsive control input to a heater element of the chemical concentration secondary gas sensor to heat the chemical concentration secondary gas sensor to an operating temperature at which the chemical concentration secondary gas sensor is capable to detect an amount of the specified first gas component in the environment.

Numbered Clause 6. The system of Numbered Clause 5, wherein the heater element of the chemical concentration secondary gas sensor is also configured as a thermal property primary gas sensor, wherein an electrical resistance or electrical conductivity of the heater element that is used for determining a change in a thermal property of the environment for providing the triggering output.

Numbered Clause 7. The system of any preceding Numbered Clause, comprising interface circuitry configured to disable the chemical concentration secondary gas sensor and reset the triggering output of the thermal property primary gas sensor when the chemical concentration secondary gas sensor indicates that a concentration of the specified first gas component falls below a threshold criterion.

Numbered Clause 8. The system of any preceding Numbered Clause, wherein the chemical concentration secondary gas sensor includes a metal oxide (MOx) gas sensor.

Numbered Clause 9. The system of any preceding Numbered Clause, wherein the thermal property of the environment is determined by monitoring an electrical resistance or an electrical conductivity of a heater element associated with at least one of the thermal property primary gas sensor or the chemical concentration secondary gas sensor.

Numbered Clause 10. The system of any preceding Numbered Clause, wherein the thermal property of the environment includes a thermal diffusivity.

Numbered Clause 11. The system of any preceding Numbered Clause, wherein the thermal property of the environment includes a heat capacity.

Numbered Clause 12. The system of any preceding Numbered Clause, wherein the thermal property of the environment includes a thermal conductivity.

Numbered Clause 13. The system of any preceding Numbered Clause, further comprising:
an ambient environment sensor configured to monitor at least one ambient environmental condition; and
interface circuitry configured to use the at least one ambient environmental condition for at least one of determining a thermal property criterion indicating a change in the gas composition of the environment or for detecting a concentration of the specified first gas component in the environment.

Numbered Clause 14. The system of Numbered Clause 13, wherein the ambient environmental condition includes an ambient temperature.

Numbered Clause 15. The system of any of Numbered Clauses 13 to 14, wherein the ambient environmental condition includes a humidity.

Numbered Clause 16. The system of any of Numbered Clauses 13 to 15, wherein the ambient environmental condition includes a gas flow.

Numbered Clause 17. The system of any of any preceding Numbered Clauses 13 to 16, wherein the ambient environmental condition includes a barometric pressure.

Numbered Clause 18. The system of any preceding Numbered Clause, further comprising at least one additional auxiliary chemical concentration sensor for detecting at least a second gas component in the environment.

Numbered Clause 19. A system for monitoring volatile gases in a battery or battery compartment environment, the system comprising:
a thermal property primary gas sensor, configured to provide a triggering output in response to determining a change in a thermal property of the environment indicating a change in a gas composition of the environment; and
a metal oxide (MOx) chemical concentration secondary gas sensor, enabled in response to the triggering output from the thermal property primary gas sensor, for detecting an amount of a specified second gas component in the environment.

Numbered Clause 20. The system of Numbered Clause 19 wherein the thermal property primary sensor monitors an electrical resistance of a heater element associated with the MOx chemical concentration secondary gas sensor to determine the change in the thermal property of the environment.

Numbered Clause 21. A method for monitoring for volatile gases in a battery or battery compartment environment, the method comprising:
measuring an electrical resistance of a MOx sensor heater element to provide an indication of a change in a composition of an ambient gas present in the environment about the MOx heater;
   triggering an increase in a temperature of the MOx heater element to an operating temperature of a corresponding MOx gas sensor for enabling gas detection, in response to the indication of the ambient gas present about the MOx heater based on the measured electrical resistance of the MOx heater element; and
determining, using the MOx sensor, a presence of a specified volatile gas in the battery or battery compartment environment.

Numbered Clause 22. The method of Numbered Clause 21 further comprising triggering corrective action after a specific volatile gas is detected.

## Claims

1. A gas monitoring system for monitoring gas in an environment, the system comprising:
a thermal property primary gas sensor, configured to provide a triggering output in response to determining a change in a thermal property of the environment indicating a change in a gas composition of the environment; and
a chemical concentration secondary gas sensor, enabled in response to the triggering output from the thermal property primary gas sensor, for detecting an amount of a specified first gas component in the environment.

2. The gas monitoring system of claim 1, wherein the chemical concentration secondary gas sensor is enabled by at least one of powering up the chemical concentration secondary gas sensor or heating a heater element of the chemical concentration secondary gas sensor to an operating temperature at which the chemical concentration secondary gas sensor is capable to detect an amount of the specified first gas component in the environment, preferably wherein the chemical concentration secondary gas sensor is at least one of powered down or operated at a standby temperature by the heater element in an absence of being enabled in response to the triggering output from the thermal property primary gas sensor, wherein the standby temperature is less than the operating temperature.

3. The gas monitoring system of claim 1 or 2, wherein the thermal property primary gas sensor is configured to detect a thermal property comprising at least one of a thermal conductivity of the environment, a heat capacity of the environment, or a thermal diffusivity of the environment, wherein the thermal property varies with a gas composition of the environment.

4. The gas monitoring system of any preceding claim, further comprising interface circuitry, receiving the triggering output from the thermal property primary gas sensor, and providing a responsive control input to a heater element of the chemical concentration secondary gas sensor to heat the chemical concentration secondary gas sensor to an operating temperature at which the chemical concentration secondary gas sensor is capable to detect an amount of the specified first gas component in the environment, preferably wherein the heater element of the chemical concentration secondary gas sensor is also configured as a thermal property primary gas sensor, wherein an electrical resistance or electrical conductivity of the heater element that is used for determining a change in a thermal property of the environment for providing the triggering output.

5. The gas monitoring system of any preceding claim, comprising interface circuitry configured to disable the chemical concentration secondary gas sensor and reset the triggering output of the thermal property primary gas sensor when the chemical concentration secondary gas sensor indicates that a concentration of the specified first gas component falls below a threshold criterion.

6. The gas monitoring system of any preceding claim, wherein the chemical concentration secondary gas sensor includes a metal oxide (MOx) gas sensor.

7. The gas monitoring system of any preceding claim, wherein the thermal property of the environment is determined by monitoring an electrical resistance or an electrical conductivity of a heater element associated with at least one of the thermal property primary gas sensor or the chemical concentration secondary gas sensor.

8. The gas monitoring system of any preceding claim, wherein the thermal property of the environment includes one or more of the following: a thermal diffusivity; a heat capacity; and a thermal conductivity.

9. The gas monitoring system of any preceding claim, further comprising:
an ambient environment sensor configured to monitor at least one ambient environmental condition; and
interface circuitry configured to use the at least one ambient environmental condition for at least one of determining a thermal property criterion indicating a change in the gas composition of the environment or for detecting a concentration of the specified first gas component in the environment.

10. The gas monitoring system of claim 9, wherein the ambient environmental condition includes one or more of an ambient temperature; a humidity; a gas flow; and a barometric pressure.

11. The gas monitoring system of any preceding claim, further comprising at least one additional auxiliary chemical concentration sensor for detecting at least a second gas component in the environment.

12. A system for monitoring volatile gases in a battery or battery compartment environment, the system comprising:
a thermal property primary gas sensor, configured to provide a triggering output in response to determining a change in a thermal property of the environment indicating a change in a gas composition of the environment; and
a metal oxide (MOx) chemical concentration secondary gas sensor, enabled in response to the triggering output from the thermal property primary gas sensor, for detecting an amount of a specified second gas component in the environment.

13. The system of claim 12, wherein the thermal property primary sensor monitors an electrical resistance of a heater element associated with the MOx chemical concentration secondary gas sensor to determine the change in the thermal property of the environment.

14. A method for monitoring for volatile gases in a battery or battery compartment environment, the method comprising:
measuring an electrical resistance of a MOx sensor heater element to provide an indication of a change in a composition of an ambient gas present in the environment about the MOx heater;
triggering an increase in a temperature of the MOx heater element to an operating temperature of a corresponding MOx gas sensor for enabling gas detection, in response to the indication of the ambient gas present about the MOx heater based on the measured electrical resistance of the MOx heater element; and
determining, using the MOx sensor, a presence of a specified volatile gas in the battery or battery compartment environment.

15. The method of claim 14, further comprising triggering corrective action after a specific volatile gas is detected.
